# EUROPÄISCHE PATENTSCHRIFT

(11) **EP 1 276 460 B1**
(45) Veröffentlichungstag und Bekanntmachung des Hinweises auf die Patenterteilung: **09.08.2006**
(21) Anmeldenummer: 01943263.2
(22) Anmeldetag: 19.04.2001
(51) Int. Cl.: A61Q 5/00, A61Q 19/00, A61K 8/97

(54) **VERWENDUNG VON ZUBEREITUNGEN ENTHALTEND EINEN EXTRAKT DER PFLANZE ARGANIA SPINOSA IN KOSMETISCHEN PFLEGEMITTELN FÜR HAARE UND HAUT**
USE OF PREPARATIONS CONTAINING AN EXTRACT OF THE PLANT ARGANIA SPINOSA IN COSMETIC CARE PRODUCTS FOR HAIR AND SKIN
UTILISATION DE PREPARATIONS CONTENANT UN EXTRAIT DE LA PLANTE ARGANIA SPINOSA DANS DES PRODUITS DE SOIN COSMETIQUES POUR LES CHEVEUX ET LA PEAU

(30) Priorität: 28.04.2000 EP 00440119
(43) Veröffentlichungstag der Anmeldung: 22.01.2003
(73) Patentinhaber: Cognis France, S.A.S., 31360 Boussens (FR)
(72) Erfinder: HENRY, Florence, F-54600 Villers-les-Nancy (FR); PAULY, Gilles, F-54000 Nancy (FR); MOSER, Philippe, F-54270 Essey-les-Nancy (FR); CHARROUF, Zoubida, B.P. 1014 Rabat R.P. (MA)
(74) Vertreter: Fabry, Bernd
(86) Internationale Anmeldenummer: PCT/EP2001/004439
(87) Internationale Veröffentlichungsnummer: WO 2001/082885

(56) Entgegenhaltungen:
- FR-A- 2 553 788
- FR-A- 2 724 663
- FR-A- 2 756 183
- STN, Karlsruhe, DE, Datenbank CAPLUS, AN=1998:646383 XP002146800
- STN, Karlsruhe, DE, Datenbank CAPLUS, AN=1998:646382 XP002146801
- STN, Karlsruhe, DE, Datenbank CAPLUS, AN=1992:630088 XP002146802
- STN, Karlsruhe, DE, Datenbank EMBASE, AN=97055617 XP002146803
- STN, Karlsruhe, DE, Datenbank EMBASE, AN=79238478 XP002146804
- STN, Karlsruhe, DE, Datenbank EMBASE, AN=79202409 XP002146805
- STN, Karlsruhe, DE, Datenbank CAPLU, AN=1974:482231 XP002146806

## Beschreibung

### Gebiet der Erfindung

Die Erfindung befindet sich auf dem Gebiet der kosmetischen Pflegestoffe und betrifft die Verwendung von Haar- und Hautpflegemittel in Kosmetik.

### Stand der Technik

Haarkosmetische Zubereitungen, wie beispielsweise Haarlacke oder Stylingmittel enthalten als Festigungsmittel synthetische Polymere, vorzugsweise vom Typ der Polyvinylpyrrolidone und Vinylacetat [vergl. **US 5637296**], die auf Keratinfasern aufziehen und ihnen den gewünschten Halt und die erhöhte Biegefestigkeit verschaffen. Nachteilig ist jedoch, dass diese Filme gerade bei mehrfacher Applizierung nach dem Trocknen leicht brüchig werden und es zusätzlich zu einer Schädigung des Haares kommen kann. Als Haarfestiger oft beschriebene ethoxylierte C12- bis C20 Fettalkohole werden in Verbindung mit nichthalogenierten, wasserlöslichen Lösungsmitteln verwendet. Diese Lösungsmittel trockenen die Haare in unerwünschter Weise aus und können zusätzlich Hautreizungen hervorrufen. **[WO 94/08554].**

Konventionelle Konditioniermittel vom Typ der Kationentenside oder Kationpolymere sind synthetische Verbindungen, die in recht hohen Konzentrationen eingesetzt werden, auf das Haar aufziehen und zum gewünschten Effekt führen. Beim Herauswaschen können diese Konditioniermittel oftmals nicht leicht entfernt werden, was bei dauerhafter Anwendung zu Schädigungen der Haare und der Kopfhaut führen kann.

Der Wunsch nach Haarkosmetischen Mitteln, die gleichzeitig die Biegefestigkeit des Haares erhöhen, den Glanz und das Volumen vermehren, jedoch bei vermehrter Applikation nicht zu einer Schädigung der Haare führen, wird vom Verbraucher immer häufiger gestellt. Weiterhin besteht ein steigendes Interesse nach neuen und wirkungsvollen Stoffen für die Kosmetik, die natürlichen Ursprungs sind und die aus nachwachsenden Rohstoffen erhalten werden können. Diese Stoffe sollen möglichst neben einer guten Konditionierung gleichzeitig einen pflegenden Effekt für das Haar und für die Haut aufweisen.

Kosmetische Zubereitungen stehen dem Verbraucher heute in einer Vielzahl von Kombinationen zur Verfügung. Dennoch besteht im Markt das Bedürfnis nach Produkten mit einem verbesserten Leistungsspektrum. Hierbei sind Hautverträglichkeit sowie der Einsatz natürlicher Produkte beim Kunden gefragt. Daneben ist es wünschenswert, durch Kombination bereits bekannter Wirkstoffe, oder durch auffinden neuer Einsatzgebiete bereits bekannter Substanzklassen deutlich bessere Produkte zu erhalten. Besonders Extrakte von Pflanzen und deren Inhaltstoffe finden immer häufiger Einsatz in der Kosmetik und Pharmazie. Es sind jedoch viele Pflanzen und ihre potentielle Wirkung noch nicht gefunden worden und viele neue Anwendungsgebiete bereits bekannter Substanzklassen sind immer wieder überraschend.

Seit langem ist bekannt, dass viele Saponine, die aus den unterschiedlichsten Pflanzen und Mikroorganismen gewonnen werden, eine anti-radikalische, analgetische und auch antiinflammatorische Wirkung zeigen. Diese Wirkung konnten Alaoui et al. auch für die aus Argania spinosa isolierten Saponine nachweisen [Alaoui K. et al.; *Annales pharmaceutiques françaises*, 1998, 56, 220-228.]. Es wurde außerdem für einige Saponine eine antibiotische und eine fungistatische Wirksamkeit gefunden. Saponine, speziell die Triterpen-Saponine sind aus einem tetra- oder pentacyclischen Triterpen-Aglykon und einer oder zwei glycosidisch gebundenen Zuckerketten aufgebaut.

In der Regel werden Saponine aufgrund ihrer ausgeprägten Schaumbeständigkeit und aufgrund des Emulgierverhaltens in Tensidmischungen eingesetzt [**DE 220448**].

Saponine aus Extrakten der Pflanze Hedera helix in Kombination mit Extrakten aus Amica Montana und Extrakten aus Colanuß werden in kosmetischen Mitteln für die Haut eingesetzt, die eine schlankmachende Wirkung zeigen **[DE 3204370].** Die einzige Anwendungsmöglichkeit dieser Kombination aus mehreren Extrakten und Saponinen aus den erwähnten Pflanzen, ist ihr Einsatz als schlankmachende Mittel mit anti-cellulitis-Wirkung.

### Beschreibung der Erfindung

Die Aufgabe der vorliegenden Patentanmeldung hat darin bestanden, Pflanzenextrakte zu finden, die einen Einsatz in der Haarbehandlung und Haarpflege ermöglichen und die aufgrund ihrer Eigenschaften z.B. den Glanz, die Kämmbarkeit in trockenem und nassem Zustand und das Volumen erhöhen bzw. verbessern.

Eine weitere Aufgabe der vorliegenden Patentanmeldung hat darin bestanden, kosmetische Zubereitungen zur Verfügung zu stellen, die Wirkstoffe aus nachwachsenden Rohstoffen enthalten und gleichzeitig vielseitig als Pflegemittel in der Kosmetik sowohl in der Haarpflege, als auch in der Hautkosmetik einsetzbar sind.

Gegenstand der Erfindung ist die Verwendung von Zubereitungen, die Saponine enthalten, welche aus der Pflanze Argania spinosa extrahiert werden, in kosmetischen pflegemitteln für Haare und/oder Haut. Überraschenderweise wurde gefunden, dass durch den Einsatz von Saponine aus Extrakten der Pflanze Argania spinosa Produkte erhalten werden, die gleichzeitig gute pflegende und schützende Eigenschaften für Haare und Haut aufweisen, sowie eine hohe Hautverträglichkeit besitzen. Die so erhaltenen Mittel zeichnen sich durch besonders gute Effekte beim Haar aus. Sie verbessern die Kämmbarkeit von trockenem und nassem Haar, erhöhen den Glanz und das Volumen und ergeben gleichzeitig eine erhöhte Biegefestigkeit am Haar, was den Einsatz in kombinierten Präparaten wie beispielsweise tägliche Anwendung von Haarfestiger am nassen sowie an gebleichtem oder dauergewelltem, geschädigtem Haar erlaubt.

Darüber hinaus wurde gefunden, dass die so erhaltenen Mittel besonders gut einsetzbar sind in der Hautpflege als schlankmachende Mittel mit anti-cellulitis Wirkung. Diese vergleichbare Wirkung mit den Saponinen aus Hedera helix, benötigt jedoch nicht den stimulierenden Effekt weiterer Pflanzenextrakte anderer Pflanzen, wie es in der DE3204370 beschrieben ist und zeigt somit einen entscheidenden Vorteil gegenüber den dort beschriebenen Mitteln.

Diese mehrfachen Einsatzgebiete der Mittel aus dem nachwachsendem Rohstoff der Pflanze Argania spinosa macht es für den Markt und für den Verbraucher sehr attraktiv. Die komplexe Aufgabe der Erfindung konnte somit durch den Einsatz der Saponine aus Argania spinosa gelöst werden.

Unter dem Begriff Pflanze im Sinne der vorliegenden Anmeldung sind sowohl ganze Pflanzen als auch Pflanzenteile (Blätter, Wurzeln, Stamm, Rinde, Blüten, Früchte, Fruchtfleisch und Samenkeme) sowie deren Gemische zu verstehen. Besonders bevorzugt zur Extraktion der Saponine im Sinne der Erfindung sind die Samenkerne der Frucht dieser Pflanze insbesondere die Extraktion des Rückstands aus den entfetteten Samenkemen.

### Argania spinosa

Die einzusetzenden Extrakte werden aus Pflanzen der Familie der Sapotaceae, speziell aus Argania spinosa gewonnen. Bei dieser Pflanze handelt es sich um einen an den Ölbaum erinnernden Baum, der überwiegend in Marokko an der Westseite des Atlasgebirges zu finden ist. Er bildet an seinen knorrigen Ästen und bedomten Zweigen Beeren von der Größe und Gestalt der Oliven mit ein bis zwei Samenkemen. Das nussartig schmeckende Öl aus den Samenkemen dient unter anderem als Speiseöl.

### Saponine

Unter Saponine sind im Sinne der Erfindung grundsätzlich alle Saponine zu verstehen, die sich aus der Pflanze Argania spinosa isolieren lassen.

Aus dem Rückstand, der bei der Ölgewinnung aus den Samenkemen von Argania spinosa anfällt, werden Saponine erhalten, die sich in der Struktur von Saponinen aus anderen Pflanzen unterscheiden [Charrouf Z., et al.; *Phytochemistry,* **1992**, 31; 2079-2086.]. Es handelt sich hier um Saponine mit der Bezeichnung Arganin A, Arganin B, Arganin C, Arganin D, Arganin E, Arganin F und Misaponin A. Aus dem Stamm der Pflanze können die einsetzbaren Saponine Arganin G, Arganin H, und Arganin J [Oulad-Ali A., et al.; J. *Nat. Prod.;* **1996,** 59, 193-195.]. Das Aglycon dieser Saponine weist die im Folgenden dargestellte Struktur (I) auf, die genannten Saponine unterscheiden sich jeweils in den Zuckereinheiten an R1 und R3 bzw. durch eine Hydroxygruppe an R2. Bei R3 handelt es sich um ein Tetrasaccharid und bei R1 jeweils um ein Mono- oder Disaccharid (z. B. 1,6-Diglukose für Arganin A und B).

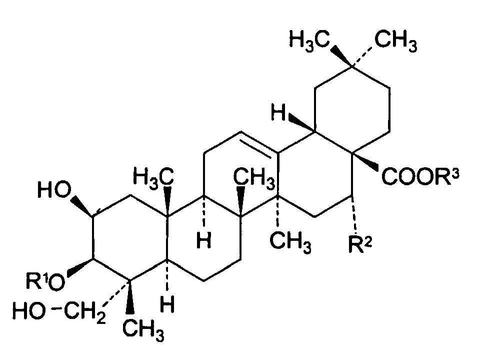

Die Saponine zeigen in toxikologischen Test an Mäusen und Ratten eine geringe Toxizität [Alaoui K., et al.; *Ann. Pharmaceutiques francaises*, **1998,** 5, 213-219.]. Im Vergleich zu anderen Saponinen wie z.B. aus Gypsophila paniculata konnten die Erfinder auch durch Tests an humanen Fibroblasten eine wesentlich geringere Toxizität nachweisen.

Die einzusetzenden Saponine entsprechen Arganin A, Arganin B, Arganin C, Arganin C, Arganin D, Arganin E, Arganin F, Misaponin A, sowie Arganin G, Arganin H, und Arganin J. Sie können als Gemisch von zwei oder mehr, oder als Reinsubstanz in der kosmetischen Zubereitung Anwendung finden. Besonders bevorzugt sind Mischungen aus Arganin A, Arganin B, Arganin C, Arganin C, Arganin D, Arganin E, Arganin F, Misaponin A, wobei die Anteile der Saponine in den Mischungen variieren können.

### Extraktion

Die Herstellung der einzusetzenden Extrakte erfolgt durch übliche Methoden der Extraktion von Pflanzen bzw. Pflanzenteilen. Bezüglich der geeigneten herkömmlichen Extraktionsverfahren wie der Mazeration, der Remazeration, der Digestion, der Bewegungsmazeration, der Wirbelextraktion, Ultraschallextraktion, der Gegenstromextraktion, der Perkolation, der Reperkolation, der Evakolation (Extraktion unter vermindertem Druck), der Diakolation und Festflüssig-Extraktion unter kontinuierlichem Rückfluß, die in einem Soxhlet-Extraktor durchgeführt wird, die dem Fachmann geläufig und im Prinzip alle anwendbar sind, sei beispielhaft auf Hagers Handbuch der **Pharmazeutischen Praxis,** (5. Auflage, Bd. 2, S. 1026-1030, Springer Verlag, Berlin-Heidelberg-New-York 1991) verwiesen. Als Ausgangsmaterial können frische oder getrocknete Pflanzen oder Pflanzenteile eingesetzt werden, üblicherweise wird jedoch von, entfetteten Pflanzen und/oder Pflanzenteilen ausgegangen, die vor der Extraktion mechanisch zerkleinert werden können. Hierbei eignen sich alle dem Fachmann bekannten Zerkleinerungsmethoden, als Beispiel sei die Zerstoßung mit einem Mörser genannt. In einer besonderen Ausführungsform werden die verwendeten Extrakte erhalten durch Extraktion des Stammes, der Wurzel, der Blätter, der Blüte oder der Früchte. Besonders bevorzugt ist die Extraktion der Samenkerne.

Als Lösungsmittel für die Durchführung der Extraktionen können vorzugsweise organische Lösungsmittel, Wasser oder Gemische aus organischen Lösungsmitteln und Wasser, insbesondere niedermolekulare Alkohole, Ester, Ketone oder halogenhaltige Kohlenwasserstoffe mit mehr oder weniger hohen Wassergehalten (destilliert oder nicht destilliert) vorzugsweise wässrig, alkoholische Lösungen einer Temperatur von über 20 °C, (im nachfolgenden als Raumtemperatur bezeichnet), verwendet werden. Besonders bevorzugt ist die Extraktion mit Wasser, Methanol, Ethanol, Aceton, Propylenglycolen, Polyethylenglycolen Ethylacetat, Dichlormethan, Trichlormethan sowie Mischungen hieraus. Die Extraktion erfolgt in der Regel bei 20 bis 100°C, bevorzugt bei 20 bis 85°C, insbesondere bei Raumtemperatur. In einer möglichen Ausführungsform erfolgt die Extraktion unter Inertgasatmosphäre zur Vermeidung der Oxidation der Inhaltsstoffe des Extraktes. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem gewünschten Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion getrockneter Pflanzen oder getrockneter Pflanzenteile gegebenenfalls entfettet, liegen im Bereich von 3 bis 20, insbesondere 4 bis 16 Gew.-%. Die vorliegende Erfindung umfasst die Erkenntnis, dass die Extraktionsbedingungen sowie die Ausbeuten der Endextrakte je nach gewünschtem Einsatzgebiet gewählt werden können. Falls gewünscht, können die Extrakte anschließend beispielsweise einer Sprüh- oder Gefriertrocknung unterworfen werden.

Die Extrakte dieser Pflanze enthalten 10 bis 100 Gew.-% Saponine, vorzugsweise 20 bis 70 Gew.-%, insbesondere 30 bis 50 Gew.-%.

Die Einsatzmenge der Pflanzenextrakte in den genannten Zubereitungen richtet sich nach der Konzentration der einzelnen Inhaltstoffe und nach der Art der Anwendungen der Extrakte. Die Gesamtmenge des Pflanzenextraktes, der in den Zubereitungen enthalten ist, beträgt in der Regel 0,01 bis 25 Gew.-%, vorzugsweise 0,03 bis 5 Gew.-%, insbesondere 0,03 bis 0,4 Gew.-% bezogen auf die Endzubereitung, mit der Maßgabe, dass sich die Mengenangaben mit Wasser und gegebenenfalls weiteren Hilfs- und Zusatzstoffen zu 100 Gew.-% addieren.

Der Gesamtanteil der Hilfs- und Zusatzstoffe kann 1 bis 50, vorzugsweise 5 bis 40 Gew.-% - bezogen auf die Endzubereitung der kosmetischen Zubereitungen - betragen. Die Herstellung der Zubereitungen kann durch übliche Kalt - oder Heißprozesse erfolgen; vorzugsweise arbeitet man nach der Phaseninversionstemperatur-Methode.

Aktivsubstanz im Sinne der Erfindung bezieht sich auf den Anteil an Substanzen sowie Hilfs- und Zusatzstoffen, die in dem kosmetischen Mittel enthaltend sind, mit Ausnahme des zusätzlich hinzugefügten Wassers.

Eine besondere Ausführungsform der vorliegenden Erfindung betrifft die vielfältige Verwendung der Saponine enthaltenden Pflanzenextrakte aus Argania spinosa beispielsweise
- in Pflegemitteln für Haare und/oder Haut, insbesondere gegen Stress und schädigenden Umwelteinflüssen.
- in Pflegemitteln zur Verbesserung der Kämmbarkeit, des Glanzes, des Volumens und der Biegefestigkeit von Haaren
- als Pflegemittel mit schlankmachender und anti-cellulitis Eigenschaften für die Haut
- in Sonnenschutzmitteln, insbesondere gegen die Schädigung von Fibroblasten und/oder Keratinocyten durch UVA-Strahlung und/oder UVB-Strahlung.

### Pflegemittel:

Als Pflegemittel im Sinne der Erfindung sind Pflegemittel für Haar und Haut zu verstehen. Diese Pflegemittel schließen unter anderem reinigende und aufbauende Wirkung für Haare und Haut ein.

Die Haarpflege hat zum Ziel, den Naturzustand des frisch nachgewachsenen Haares möglichst lange zu erhalten bzw. bei Schädigung wiederherzustellen. Merkmale natürlichen gesunden Haares sind seidiger Glanz, geringe Porosität, spannkräftige und dabei weiche Fülle und angenehm glattes Gefühl (guter "Griff"). Die Pflegemittel wirken glättend auf das Haar, sie verbessern die Kämmbarkeit und verbessern Volumen und Glanz. Durch die Anwendung der Pflegemittel erhöht sich die Biegefestigkeit des Haares, so dass die natürliche Spannkraft des Haares erhöht wird bzw. die Spannkraft von geschädigtem Haar wieder hergestellt wird.

Die Zubereitungen zeigen darüber hinaus eine hervorragende hautpflegende Wirkung bei gleichzeitig hoher Hautverträglichkeit. Außerdem zeigen sie eine gute Stabilität, insbesondere gegenüber oxidativer Zersetzung der Produkte.

Diese Pflegemittel schließen schlankmachende und anti-cellulitis Eigenschaften für die Haut ein.

In einer besondern Ausführungsform werden die Extrakte in Sonnenschutzmitteln verwendet. Sie zeigen insbesondere eine Aktivität gegen UVB Strahlung an Keratinozyten und gegen UVA Strahlung an Fibroblasten und werden bevorzugt gegen die Schädigung der Hautzellen durch UVB und/oder UVA-Strahlung verwendet.

Beispiele für kosmetische Produkte sind in ihren Formulierungen in den Tabelle 7 bis 10 beschrieben.

### Gewerbliche Anwendbarkeit

### Kosmetische Zubereitungen

Die Zubereitungen können zur Herstellung von kosmetischen Zubereitungen, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/Fett-Massen, Stiftpräparaten, Pudern oder Salben dienen. Diese Mittel können ferner als weitere Hilfs- und Zusatzstoffe milde Tenside, Ölkörper, Emulgatoren, Perlglanzwachse, Konsistenzgeber, Verdikkungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Äcyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quatemierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217** verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, lsostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂₋Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. **DE 19756377 A1**), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈₋Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestem mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
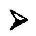 ➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispiels-weise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquatemierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B.

Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgener und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte VinylpyrrolidonNinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quatemierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der FR 2252840 A sowie deren vemetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/ Acrylat-Copolymere, Octylacrylamid/Methylmeth-acrylat/tertButylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosm.Toil. 108, 95 (1993)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der **EP 0693471 B1** beschrieben;
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexyl-ester;
➢ Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der **EP 0694521 B1** beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtem wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** sowie **Parf.Kosm. 3, 11 (1999)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4'-Trichlor-2'-hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

### Parfümöle

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedem-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

### Farbstoffe

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

### Beispiele

### 1. Beispiel: Extraktion der Pflanzensamenkerne

0,2 kg entfettete Argania spinosa Samenkerne wurden in ein Glasgefäß überführt und mit 2 l 80 Gew.-% igem wäßrigem Ethanol aufgegossen. Die Mischung wurde bei Raumtemperatur für zwei Stunden gerührt und die Feststoffe über Filtration abgetrennt. Das Ethanol aus dem Filtrat des Rohextraktes wurde im Vakuum (2000 - 2670 Pa, 15 bis 20 Torr) entfernt. Der verbleibende wässrige Extrakt wurde erneut mit n-Butanol extrahiert. Das Butanol wurde ebenfalls im Vakuum abgetrennt und der wässrige Rest lyophilisiert. Bezogen auf das Gewicht der Pflanzensamenkerne erhielt man 0,5 Gew.% Saponine.

### 2. Beispiel: Sensorische Aktivität auf unterschiedlichen Haartypen

Die Bewertung der Modifikation sensorischer Eigenschaften an menschlichen Haaren im Vergleich von geschädigten (gebleicht oder dauergewelltes Haar) zu ungeschädigten (Kontrollhaare) nach der Behandlung mit Extrakten der Pflanze Argania spinosa wurde an standardisierten Haarsträhnen (15 cm Länge und 3 g Gewicht) durchgeführt. Als Standard und Placebo diente eine wässrige Natrium-Lauryl-Sulfat Lösung (15 % w/v). Die Proben der Pflanzenextrakte wurden in einer Konzentration von 1,5 Gew.-% in die Natrium-Lauryl-Sulfat Lösung eingearbeitet und getestet. Die Behandlungseffekte wurden an drei unterschiedlichen Haartypen untersucht:
a) Kontroll-Haar: die Strähnen wurden mit einer wässrigen Natrium-Lauryl-Sulfat Lösung (15 Gew.-%), gewaschen.
b) Gebleichtes Haar: die Strähnen wurden mit einem Bleich-Schampoo, welches 6 % H₂O₂ und Ammoniak enthält (Eclair clair der Firma L'Oréal), 30 min. behandelt und anschließend mit der wässrigen Natrium-Lauryl-Sulfat Lösung (15Gew.-%) gewaschen. Dieser Bleichvorgang wurde zweimal wiederholt.
c) Dauergewelltes Haar: die Strähnen wurden mit einer Natrium-mercaptoacetat Lösung (6 % w/v, pH = 6) 20 min. behandelt, ausgespült und anschließend 10 min mit einer Lösung H₂O₂ (pH = 3) versetzt. Nach dem erneuten ausspülen dieser Lösung wurde mit wässriger Natrium-Lauryl-Sulfat Lösung (15 Gew.-%) gewaschen. Dieser Zyklus der permanenten Welle wurde zweimal wiederholt.

Die so präparierten Haarsträhnen wurden 3 min in die Lösung mit der jeweiligen Testsubstanz gehalten und anschließend 1 min ausgespült. Nach dem Spülen wurden die Haarsträhnen gekämmt und ihre Nasskämmbarkeit getestet. Die Strähnen wurden bei Raumtemperatur getrocknet. Die sensorischen Tests wurden am trockenem Haar 24 h nach der Behandlung mit den Extrakten durchgeführt.

Am trockenem Haar wurden folgende Eigenschaften bestimmt: Kämmbarkeit, Geschmeidigkeit und Weichheit, Statische Aufladung, Volumen und Glanz.

In der Tabelle finden sich die Ergebnisse der sensorischen Tests an nassem und an trockenem Haar. Die sensorischen Eigenschaften sind im Vergleich zu standardisierten Haarsträhnen zu lesen. Je höher die angegebene Zahl, desto besser ist die Bewertung der jeweiligen sensorischen Eigenschaft.

**Tabelle 1:**

| **Sensorische Eigenschaften menschlicher Haarsträhnen nach der Behandlung mit Extrakten der Pflanze Argania spinosa im Vergleich zu ungeschädigten Haarsträhnen** | | | |
|---|---|---|---|
| Parameter | Kontroll-Haar | Gebleichtes Haar | Dauergewelltes Haar |
| Kämmbarkeit an nassem Haar | 0 | 0,5 | 0 |
| Kämmbarkeit an trockenem Haar | 0,5 | 0 | 0,5 |
| Geschmeidigkeit und Weichheit | 0,5 | 0,5 | 0,5 |
| Statische Aufladung | 0 | 0 | 0 |
| Volumen | 0,5 | 0,5 | 0 |
| Glanz | 0,5 | 0,5 | 0 |

Die Ergebnisse der Test zeigen, dass ein Extrakt der Pflanze Argania spinosa die sensorischen Eigenschaften an menschlichem Haar verbessert. Die statische Aufladung ergab nach Behandlung mit dem Pflanzenextrakt keine Veränderung.

An gebleichtem Haar konnte eine Verbesserung der Kämmbarkeit an nassem Haar und des Volumens und des Glanzes verzeichnet werden. Für dauergewelltes Haar erhöhte sich die Kämmbarkeit an trockenen Haaren und die Weichheit. Für das Kontrollhaar konnte bis auf die Kämmbarkeit an nassem Haar und die statische Aufladung durchweg ein positiver Effekt verzeichnet werden. Die statische Aufladung zeigte bei keinem der verwendeten Haartypen eine Veränderung.

### 3. Beispiel: Lipolyseaktivität an humanen Adipocyten

Hintergrund: Lipolyse ist die Bezeichung für den körpereigenen Abbau von Fetten, die in den Adipocyten (Fettzellen) als Reserven vorhanden sind. Diese werden zu kleineren molekularen Bruchstücken, den Fettsäuren und dem Glycerin enzymatisch durch Lipasen gespalten. Die freien Fettsäuren werden dann von den Muskelzellen zur Energiegewinnung genutzt.

Methode: Die Adipocyten wurden aus humanem subkutanem Gewebe isoliert, wie es der allgemeinen Technik nach Rodbell entspricht. Der Extrakt nach Beispiel 1 bzw. die Vergleichssubstanzen werden im Referenzmedium gelöst und anschließend 90 Minuten bei 37 °C mit den isolierten Adipocyten in Kontakt gebracht. Es wurde je eine Adipocyten-Preparationen untersucht. Die prozentuale Erhöhung an freigesetztem Glycerin wurde spectrophotometrisch im Überstand des Mediums nach der Methode von Carpéné et al. bestimmt. Als Referenz wurde das Medium ohne zu untersuchende Substanz verabreicht und gleich 0 gesetzt.

**Tabelle 2: Lipolyseaktivität an humanen Adipocyten**

| Substanz | Konzentration in Gew.-% | %- Erhöhung an freigesetztem Glycerin |
|---|---|---|
| Saponin Extrakt nach Beispiel 1 | 0,03 | 17 |
| Saponin Extrakt nach Beispiel 1 | 0,1 | 66 |
| Saponin Extrakt nach Beispiel 1 | 0,3 | 183 |
| Caffein | 30 mM | 181 |

Die Ergebnisse der Untersuchungen belegen, dass die Saponine aus den Samenkernen der Argania spinosa eine von der Konzentration abhängige Lipolyseaktivität bei humanen Adipocyten in vitro zeigen. Diese Ergebnisse belegen die potentielle Wirkung als schlankmachende Substanzen auf der Haut bzw. die anti-cellulitis Wirkung.

### 4. Bespiel: Test an humanen Fibroblasten zu Toxizität und Wachstumsfaktor Aktivität

Das Ziel dieser Tests ist der Nachweis einer regenerierenden und revitalisierenden Aktivität von Extrakten aus Argania spinosa an humanen Fibroblastenkulturen in vitro. Der Toxizitätstest erlaubt die Untersuchung der Konzentration an Testsubstanz, mit der effiziente Test durchgeführt werden können. Die Toxizitäts- und Wachstumsfaktortests wurden durchgeführt an humanen Fibroblasten zur Untersuchung der regenerativen Aktivität von Argania spinosa Extrakten und zur Untersuchung einer Wachstumsfaktor vergleichbaren Aktivität.

Methode 1: Toxizitäts- und Zellwachstumstest: Humane Fibroblasten wurden in einem definiertem Nährmedium (DMEM = Dulbecco Minimum Essential Medium, Firma Life Technologie Sarl) mit 10 Gew.-% fötalem Kälberserum angeimpft und für 24 h bei 37°C in einer 5 %igen CO₂-Atmosphäre inkubiert. Anschließend wurde das Nährmedium mit fötalem Kälberserum durch ein Nährmedium aus DMEM ohne fötalem Kälberserum ausgetauscht. Zu diesem Nährmedium wurden unterschiedliche Konzentrationen an Aktivsubstanz in Form der Extrakte aus Argania spinosa nach Beispiel 1 gegeben. Zum Vergleich wurde als Kontrolle eine Testreihe von humanen Fibroblasten ohne Aktivsubstanz inkubiert. Nach einer drei tägigen Inkubation der Fibroblasten im Nährmedium wurde das Wachstum und die Stoffwechselaktivität beurteilt, indem die Zellen mittels eines Partikelzählers ausgezählt wurden und indem der intrazelluläre Anteil an ATP nach der Methode von Vasseur (*Joumal francais Hydrologie,* 1981, 9, 149-156.) und der Anteil an Zellproteinen nach der Methode von Bradford (*Anal. Biochem.,* **1976,** 72*, ,* 248-254) bestimmt wurde. ATP bzw. Adenosintriphosphat ist ein Energieüberträger und wird hauptsächlich durch die Mitochondrien produziert. Die Zellen benötigen ATP für die Aktivität vieler Enzyme. Die Bestimmung des Anteils an Proteinen gibt einen Hinweis auf den Anteil an synthetisierten Makromolekülen wie Enzymen, Proteinen, Makromolekülen der Dermis, welche für den Aufbau und Erhalt des Gewebes notwendig sind.

**Tabelle 3: Toxizitätstest an humanen Fibroblasten**

| EC50 (in Gew.-%) | Proteinanteil | ATP-Anteil |
|---|---|---|
| Extrakt nach Beispiel 1 | 0,0265 % | 0,0204 % |

Der Toxizitätstest zeigt, dass ein Extrakt nach Beispiel 1 eine effektive Konzentration (EC50-Wert) von 0,0265 Gew.-% bezogen auf den Proteinanteil und einen EC50 Wert von 0,0204 Gew.-% bezogen auf den Anteil an ATP besitzt. Aus diesen Ergebnissen zeigt sich eine geringe Toxizität an humanen Fibroblasten in vitro.

**Tabelle 4: Erhöhung des Proteinanteils und des ATP-Anteils in humanen Fibroblasten nach Inkubation mit Extrakten nach Beispiel 1**

| Substanz | Konzentration Gew.% | Anteil der Proteine in % | Anteil an ATP in % |
|---|---|---|---|
| Kontrolle | | 100 | 100 |
| Extrakt nach Beispiel 1 | 0,001 | 105 | 119 |
| | 0,003 | 113 | 113 |
| | 0,01 | 102 | 93 |

Bei Konzentrationen von 0,001 bis 0,01 Gew.-% Extrakt nach Beispiel 1 erhielt man eine Erhöhung des Anteils an ATP zwischen 13 und 19 % im Vergleich zur Kontrolle. Der Anteil an Zellproteinen erhöhte sich bei Konzentrationen von 0,001 bis 0,01 Gew.-% Extrakt nach Beispiel 1 zwischen 2 und 13 % im Vergleich zur Kontrolle.

Methode 2: Verbesserung der Überlebensfähigkeit Die Test wurden durchgeführt an humanen Fibroblasten. Der Test ermöglicht auf den ruhenden Zellen eine gewisse Anzahl von Parametern mengenmäßig zu bestimmen. Die Kultivierung der Zellen entspricht der Kultivierung aus der Methode 1, ausgenommen der Inkubationszeit. Die Inkubationszeit für diese Test betrugen 72 h. Die Überlebensfähigkeit wurde beurteilt über den Anteil an MTT und den Anteil an GSH. Die Mitochondriale Aktivität wird bestimmt durch MTT, einem Farbstoff welcher durch das Enzym Succinat-Dehydrogenase in Formazan umgewandelt wird. Dieser Test wurde durchgeführt nach der Methode von Denizot F. und Lang R. beschrieben in: "Rapid colorimetric assay for cell growth and survival ; J. Immunol. Methods, 89, 271-277, 1986". Glutathion (GSH) ist ein kleines Peptid aus drei Aminosäuren, welches die Zelle vor oxidativen Stress oder schädigenden Umwelteinflüssen wie z.B. Schwermetalle schützen kann. Der Anteil an GSH wurde fluorimetrisch nach der Methode von Hissin PJ. und Hilf R. beschrieben in: "A fluorometric method for determination of oxydised and reduced Glutathione in tissus ; Analytical Biochemistry, 74 : 214-226, 1976" durchgeführt. Die Test wurden dreifach durchgeführt und dann zweimal wiederholt, so dass es sechs Ergebnisse pro Extrakt gab, die jeweils gemittelt wurden. Die Ergebnisse wurden ermittelt in Prozent im Vergleich zur Kontrolle.

**Tabelle 5: Parameter zur Bestimmung der Verbesserung der Überlebensfähigkeit**

| | Konzentration an Extrakt in Gew.-% | Anteil an MTT in % | Anteil an Proteinen in % | Anteil an GSH |
|---|---|---|---|---|
| Kontrolle | 0 | 100 | 100 | 100 |
| Extrakt nach Beispiel 1 | 0,001 | 106 | 96 | 119 |
| | 0,003 | 114 | 102 | 119 |
| | 0,001 | 110 | 87 | 184 |

Die Ergebnisse zeigen, dass ein Extrakt nach Beispiel 1 aus Argania spinosa die Synthese von Glutathion erheblich stimuliert. Diese Eigenschaft macht den Einsatz von Extrakten aus Argania spinosa als revitalisierende und reaktivierende Mittel in kosmetischen und/oder pharmazeutischen Mitteln möglich. Eine Verwendung als hautverjüngendes Mittel wird ebenfalls möglich.

### 5. Beispiel: Zellschutzwirkung gegen UVB an in vitro gezüchteten menschlichen Keratinozyten

Hintergrund: UVB-Strahlen lösen durch Aktivierung eines Enzyms, nämlich Phospholipase A2 oder PLA2, welche Arachidonsäure aus den Phospholipiden der Plasmamembran entfernt, eine Entzündung (Erythem, Ödem) aus. Arachidonsäure ist die Vorstufe der Prostaglandine, die eine Entzündung und eine Zellmembranschädigung verursachen; die Prostaglandine E2 (= PGE2) werden durch die Cyclooxygenase gebildet.

Methode: Der Effekt von UVB-Strahlung wurde an Keratinocyten in vitro untersucht indem die Freisetzung des Cytoplasaenzyms LDH (Lactat Dehydrogenase) bestimmt wurde. Dieses Enzym dient als Marker für eine Zellschädigung.

Zur Durchführung der Tests wurde ein definiertes Medium, das fötales Kälberserum enthält, mit den Keratinozyten beimpft und der Extrakt nach Beispiel 1 (mit Saline-Lösung verdünnt) 72 Stunden nach dem Beimpfen zugegeben.

Die Keratinozyten wurden sodann mit einer UVB-Dosis bestrahlt (30 mJ/cm² - Röhren: DUKE FL40E).

Nach weiterer 1 tägiger Inkubation bei 37 °C und bei 5 % CO₂ wurde der LDH-Gehalt im Überstand bestimmt. Der Gehalt von LDH- (Lactatdehydrogenase) wurde mittels einer Enzymreaktion bestimmt (verwendetes kit zur Untersuchung des LDH Gehaltes von der Firma Roche). Die Anzahl adhärenter Keratinozyten wird (nach Trypsinbehandlung) mit einem Partikelzählgerät bestimmt.

**Tabelle 6 Zellschutzwirkung eines Extraktes von Argania spinosa gegen UVB-Strahlen; Ergebnisse in % bezogen auf die Kontrolle, Mittelwert aus 2 Versuchen, jeder mit zwei Wiederholungen**

| Extrakt nach Beispiel 1 | Anzahl Keratinocyten | Gehalt an freigesetzte LDH |
|---|---|---|
| Kontrolle ohne UV | 100 | 0 |
| UVB (30mj/cm2) | 49 | 100 |
| UVB + Extrakt 0,03 % | 90 | 44 |

Die Ergebnisse dieser Tests belegen, dass ein Extrakt der Pflanze Argania spinosa den Effekt von UVB-Strahlung auf die Anzahl an Keratinocyten und auf den Gehalt an freigesetzte LDH reduziert. Die beschriebenen Extrakte zeigen demnach die Fähigkeit, die durch UVB-Strahlung hervorgerufene Schädigung an Zellmembranen zu reduzieren.

### 6. Beispielrezepturen kosmetischer Mittel mit Argania spinosa Extrakten

Die gemäß Beispiel 1 gewonnenen Saponine aus Argania spinosa Extrakt wurden in den folgenden Rezepturen K1 bis K21 sowie 1 bis 25 eingesetzt. Die so hergestellten kosmetischen Mittel zeigten gegenüber den Vergleichsrezepturen V1, V2 und V3 sehr gute hautpflegende Eigenschaften bei gleichzeitig guter Hautverträglichkeit. Darüber hinaus sind die Mittel stabil gegen oxidative Zersetzung.

**Tabelle 7: Softcreme Rezepturen K1 bis K7**

| (Alle Angaben in Gew.-% bez. auf das kosmetische Mitteln) | | | | | | | | |
|---|---|---|---|---|---|---|---|---|
| INCI Bezeichnung | K1 | K2 | K3 | K4 | K5 | K6 | K7 | V1 |
| Glyceryl Stearate (and) Ceteareth-12/20 (and) Cetearyl Alcohol (and) Cetyl Palmitate | 8,0 | 8,0 | 8,0 | 8,0 | 8,0 | 8,0 | 8,0 | 8,0 |
| Cetearyl Alcohol | 2,0 | 2,0 | 2,0 | 2,0 | 2,0 | 2,0 | 2,0 | 2,0 |
| Dicaprylyl Ether | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetearyllsononanoate | 3,0 | 3,0 | 3,0 | 3,0 | 3,0 | 3,0 | 3,0 | 3,0 |
| Glycerin (86 Gew.-%ig) | 3,0 | 3,0 | 3,0 | 3,0 | 3,0 | 3,0 | 3,0 | 3,0 |
| Saponin Extrakt nach Beispiel 1 | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

**Tabelle 8: Nachtcremerezepturen K8 bis K14**

| (Alle Angaben in Gew.-% bez. auf das kosmetische Mitteln) | | | | | | | | |
|---|---|---|---|---|---|---|---|---|
| INCI Bezeichnung | K8 | K9 | K10 | K11 | K12 | K13 | K14 | V2 |
| Polyglyceryl-2 Dipolyhydroxystearate | 4,0 | 4,0 | 4,0 | 4,0 | 4,0 | 4,0 | 4,0 | 5,0 |
| Polyglyceryl-3 Diisostearate | 2,0 | 2,0 | 2,0 | 2,0 | 2,0 | 2,0 | 2,0 | 2,0 |
| Cera Alba | 2,0 | 2,0 | 2,0 | 2,0 | 2,0 | 2,0 | 2,0 | 2,0 |
| Zinc Stearate | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetaeryllsononanoate | 8,0 | 8,0 | 8,0 | 8,0 | 8,0 | 8,0 | 8,0 | 8,0 |
| Dicaprylyl Ether | 5,0 | 5,0 | 5,0 | 5,0 | 5,0 | 5,0 | 5,0 | 5,0 |
| Magnesiumsulfate | 1,0 | 1,0 | 1,0 | 1,0 | 1,0 | 1,0 | 1,0 | 1,0 |
| Glycerin (86 Gew.-%ig) | 5,0 | 5,0 | 5,0 | 5,0 | 5,0 | 5,0 | 5,0 | 5,0 |
| Saponin Extrakt nach Beispiel 1 | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

**Tabelle 9: W/O Bodylotion Rezepturen K15 bis K21**

| (Alle Angaben in Gew.-% bez. auf das kosmetische Mitteln) | | | | | | | | |
|---|---|---|---|---|---|---|---|---|
| INCI-Bezeichnung | K15 | K16 | K17 | K18 | K19 | K20 | K21 | V3 |
| PEG-7 Hydrogenated Castor Oil | 7,0 | 7,0 | 7,0 | 7,0 | 7,0 | 7,0 | 7,0 | 7,0 |
| Decyl Oleate | 7,0 | 7,0 | 7,0 | 7,0 | 7,0 | 7,0 | 7,0 | 7,0 |
| Cetearyl Isononanoate | 7,0 | 7,0 | 7,0 | 7,0 | 7,0 | 7,0 | 7,0 | 7,0 |
| Glycerin (86 Gew.-%ig) | 5,0 | 5,0 | 5,0 | 5,0 | 5,0 | 5,0 | 5,0 | 5,0 |
| MgSO₄ · 7 H₂O | 1,0 | 1,0 | 1,0 | 1,0 | 1,0 | 1,0 | 1,0 | 1,0 |
| Saponin Extrakt nach Beispiel 1 | 1,5 | 1,5 | 1,5 | 1,5 | 1,5 | 1,5 | 1,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

| | | | | | | | | |
|---|---|---|---|---|---|---|---|---|
| ¹⁾ Desoxyribonucleinsäure: Molekuargewicht ca. 70000, Reinheit (bestimmt durch spektro-photometrische Messung der Absorption bei 260 nm sowie 280 nm): mindestens 1,7. | | | | | | | | |

**Tabelle 10: Rezepturen**

| **Kosmetische Zubereitungen Conditioner (Wasser, Konservierungsmittel ad 100 Gew.-%)** | | | | | | |
|---|---|---|---|---|---|---|
| **Zusammensetzung (INCI)** | **1 Gew.-%** | **2 Gew.-%** | **3 Gew.-%** | **4 Gew.-%** | **5 Gew.-%** | **6 Gew.-%** |
| **Dehyquart® A** | 4,0 | 4,0 | | | 3,0 | |
| Cetrimonium Chloride | | | | | | |
| **Dehyquart L® 80** | | | 1,2 | 1,2 | | 1,0 |
| Dicocoylmethylethoxymonium Methosulfate (and) Propylenglycol | | | | | | |
| **Eumulgin® B2** | 0,8 | | - | 0,8 | - | 1,0 |
| Ceteareth-20 | | | | | | |
| **Eumulgin® VL 75** | - | 2,0 | 2,0 | - | 0.8 | - |
| Lauryl Glucoside (and) Polyglyceryl-2 Polyhydroxystearate (and) Glycerin | | | | | | |
| **Lanette® 0** | 3,0 | 3,0 | 3,0 | 3,0 | 3,0 | 3,0 |
| Cetearyl Alcohol | | | | | | |
| **Cutina® GMS** | - | 0,5 | - | 0,5 | - | 1,0 |
| Glyceryl Stearate | | | | | | |
| **Lamesoft® PO 65** | | - | 3,0 | - | - | 3,0 |
| Coco-Glucosid (and) Glyceryl Oleate | | | | | | |
| **Cetiol® J 600** | - | 0,5 | - | 1,0 | - | 1,0 |
| Oleyl Erucate | | | | | | |
| **Eutanol® G** | - | - | 1,0 | - | - | 1,0 |
| Octyldodecanol | | | | | | |
| **Nutrilan® Keratin W** | 5,0 | - | - | 2,0 | - | - |
| Hydrolyzed Keratin | | | | | | |
| **Generol® 122 N** | - | - | - | - | 1,0 | 1,0 |
| Soja Sterol | | | | | | |
| **Saponin Extrakt nach Beispiel 1** | 1,0 | 1,0 | 1,0 | 1,0 | 1,0 | 1,0 |
| **Copherol® 1250** | - | - | 0,1 | 0,1 | - | - |
| Tocopheryl Acetate | | | | | | |

| | | | | | | |
|---|---|---|---|---|---|---|
| (1-4) Haarspülung, (5-6) Haarkur | | | | | | |

**Tabelle 10: Rezepturen**

| **Kosmetische Zubereitungen Conditioner (Wasser, Konservierungsmittel ad 100 Gew.-%)** | | | | |
|---|---|---|---|---|
| **Zusammensetzung (INCI)** | **7 Gew.-%** | **8 Gew.-%** | **9 Gew.-%** | **10 Gew.-%** |
| **Texapon® NSO** | 38,0 | 38,0 | 25,0 | - |
| Sodium Laureth Sulfate | | | | |
| **Texapon® SB 3** | - | - | 10,0 | - |
| Disodium Laureth Sulfosuccinate | | | | |
| **Plantacare® 818** | 7,0 | 7,0 | 6,0 | - |
| Coco Glucosides | | | | |
| **Plantacare® PS 10** | - | - | - | 20,0 |
| Sodium Laureth Sulfate (and) Coco Glucosides | | | | |
| **Dehyton® PK 45** | - | - | 10,0 | |
| Cocamidopropyl Betaine | | | | |
| **Lamesoft® PO 65** | 3,0 | | | 4,0 |
| Coco-Glucosid (and) Glyceryl Oleate | | | | |
| **Lamesoft® LMG** | - | 5,0 | - | |
| Glyceryl Laurate (and) Potassium Cocoyl Hydrolyzed Collagen | | | | |
| **Euperlan® PK 3000 AM** | - | 3,0 | 5,0 | 5,0 |
| Glycol Distearate (and) Laureth-4 (and) Cocamidopropyl Betaine | | | | |
| **Saponin Extrakt nach Beispiel 1** | 1,0 | 1,0 | 1,0 | 1,0 |
| **Arlypon® F** | 3,0 | 3,0 | 1,0 | - |
| Laureth-2 | | | | |
| **Sodium Chloride** | - | 1,5 | - | 1,5 |

| | | | | |
|---|---|---|---|---|
| (7-8) Duschbad, (9) Duschgel, (10) Waschlotion | | | | |

**Tabelle 10**

| **Kosmetische Zubereitungen Duschbad "Two in One" (Wasser, Konservierungsmittel ad 100 Gew.-%) - Fortsetzung** | | | | |
|---|---|---|---|---|
| **Zusammensetzung (INCI)** | 11 | 12 | 13 | 14 |
| **Texapon® NSO** | 30,0 | 25,0 | | 25,0 |
| Sodium Laureth Sulfate | | | | |
| **Plantacare® 818** | | | | 8,0 |
| Coco Glucosides | | | | |
| **Plantacare® 2000** | | 8,0 | | |
| Decyl Glucoside | | | | |
| **Plantacare® PS 10** | | | 20,0 | |
| Sodium Laureth Sulfate (and) Coco Glucosides | | | | |
| **Dehyton® PK 45** | | 10,0 | 10,0 | |
| Cocamidopropyl Betaine | | | | |
| **Lamesoft® PO 65** | 5,0 | | | |
| Coco-Glucosid (and) Glyceryl Oleate | | | | |
| **Lamesoft® LMG** | | 5,0 | 5,0 | |
| Glyceryl Laurate (and) Potassium Cocoyl Hydrolyzed Collagen | | | | |
| **Gluadin® WQ** | 3,0 | | | |
| Laurdimonium Hydroxapropyll Hydrolyzed Wheat Protein | | | | |
| **Gluadin® WK** | | | | |
| Sodium Cocoyl Hydrolyzed Wheat Protein | | | | |
| **Euperlan® PK 3000 AM** | 5,0 | 3,0 | 4,0 | - |
| Glycol Distearate (and) Laureth-4 (and) Cocamidopropyl Betaine | | | | |
| **Panthenol** | 0,5 | - | - | 0,5 |
| **Saponin Extrakt nach Beispiel 1** | 1,0 | 1,0 | 1,0 | 1,0 |
| **Arlypon® F** | 2,6 | 1,6 | - | 1,0 |
| Laureth-2 | | | | |
| **Sodium Chloride** | - | - | - | - |

**Tabelle 10**

| **Kosmetische Zubereitungen Shampoo (Wasser, Konservierungsmittel ad 100 Gew.-%) - Fortsetzung** | | | | | | |
|---|---|---|---|---|---|---|
| **Zusammensetzung (INCI)** | **15** | **16** | **17** | **18** | **19** | **20** |
| **Texapon® NSO** | 30,0 | | | 30,0 | 25,0 | |
| Sodium Laureth Sulfate | | | | | | |
| **Texapon® K 14 S** | | 30,0 | | | | 30,0 |
| Sodium Myreth Sulfate | | | | | | |
| **Texapon® SB 3** | | 10,0 | | | | |
| Disodium Laureth Sulfosuocinate | | | | | | |
| **Plantacare® 818** | 4,0 | | | | | |
| Coco Glucosides | | | | | | |
| **Plantacare® 2000** | | 4,0 | | | | |
| Decyl Glucoside | | | | | | |
| **Plantacare® PS 10** | | | 20,0 | | | |
| Sodium Laureth Sulfate (and) Coco Glucosides | | | | | | |
| **Dehyton® PK 45** | 5,0 | | | 10,0 | | 10,0 |
| Cocamidopropyl Betaine | | | | | | |
| **Gluadin® WK** | | | | | 8,0 | |
| Sodium Cocoyl Hydrolyzed Wheat Protein | | | | | | |
| **Lamesoft® PO 65** | - | - | - | - | 2,0 | 2,0 |
| Coco-Glucosid (and) Glyceryl Oleate | | | | | | |
| **Nutrilan® Keratin W** | 5,0 | - | - | - | | - |
| Hydrolyzed Keratin | | | | | | |
| **Gluadin® W 40** | - | 2,0 | - | 2,0 | - | - |
| Hydrolyzed Wheat Protein | | | | | | |
| **Euperlan® PK 3000 AM** | - | - | - | 3,0 | 3,0 | - |
| Glycol Distearate (and) Laureth-4 (and) Cocamidopropyl Betaine | | | | | | |
| **Panthenol** | - | - | - | - | - | 0,2 |
| **Saponin Extrakt nach Beispiel 1** | 1,0 | 1,0 | 1,0 | 1,0 | 1,0 | 1,0 |
| **Arlypon® F** | 1,5 | - | - | - | - | - |
| Laureth-2 | | | | | | |
| **Sodium Chloride** | - | 1,6 | 2,0 | 2,2 | - | 3,0 |

**Tabelle 10**

| **Kosmetische Zubereitungen Schaumbad (Wasser, Konservierungsmittel ad 100 Gew.-%) - Fortsetzung 2** | | | | | |
|---|---|---|---|---|---|
| **Zusammensetzung** Bezeichnung nach INCI | **21** | **22** | **23** | **24** | **25** |
| **Texapon® NSO** | - | 30,0 | 30,0 | - | 25,0 |
| Sodium Laureth Sulfate | | | | | |
| **Plantacare® 818** | - | 10,0 | - | | - 20,0 |
| Coco Glucosides | | | | | |
| **Plantacare® PS 10** | 22,0 | - | 5,0 | 22,0 | - |
| Sodium Laureth Sulfate (and) Coco Glucosides | | | | | |
| **Dehyton® PK 45** | 15,0 | 10,0 | 15,0 | 15,0 | 20,0 |
| Cocamidopropyl Betaine | | | | | |
| **Monomuls® 90-O 18** | 0,5 | | | | |
| Glyceryl Oleate | | | | | |
| **Lamesoft® PO 65** | | 3,0 | | 3,0 | |
| Coco-Glucosid (and) Glyceryl Oleate | | | | | |
| **Cetiol® HE** | | | 2,0 | | 2,0 |
| PEG-7 Glyceryl Cocoate | | | | | |
| **Nutrilan® I-50** | 5,0 | | | | |
| Hydrolyzed Collagen | | | | | |
| **Gluadin® W 40** | | 5,0 | | 5,0 | |
| Hydrolyzed Wheat Gluten | | | | | |
| **Gluadin® WK** | | | | 7,0 | |
| Sodium Cocoyl Hydrolyzed Wheat Protein | | | | | |
| **Euperlan® PK 3000 AM** | 5,0 | - | - | 5,0 | - |
| Glycol Distearate (and) Laureth-4 (and) Cocamidopropyl Betaine | | | | | |
| **Arlypon® F** | | | 1,0 | | |
| Laureth-2 | | | | | |
| **Sodium Chloride** | 1,0 | | 1,0 | | 2,0 |
| **Saponin Extrakt nach Beispiel 1** | 1,0 | 1,0 | 1,0 | 1,0 | 1,0 |

## Patentansprüche

1. Verwendung von Zubereitungen, die Saponine aus einem Extrakt der Pflanze Argania spinosa enthalten, in kosmetischen Pflegemitteln für Haare und/oder Haut.

2. Verwendung nach Anspruch 1 zur Verbesserung der Kämmbarkeit, des Glanzes, des Volumens und der Biegefestigkeit von Haaren.

3. Verwendung nach Anspruch 1 als schlankmachende und Anti-Cellulites-Pflegemittel für die Haut.

4. Verwendung nach Anspruch 1 in Sonnenschutzmitteln.

5. Verwendung nach einem der Ansprüche 1 bis 4, wobei der Extrakt erhalten wird durch Extraktion von Pflanzenteilen, ausgewählt aus der Gruppe, die gebildet wird aus den Blättern, den Wurzeln, dem Stamm, der Rinde, den Blüten, den Früchten, dem Fruchtfleisch und den Samenkernen.

6. Verwendung nach einem der Ansprüche 1 bis 4, wobei der Extrakt erhalten wird durch Extraktion der Samenkerne und/oder der entfetteten Samenkerne aus der Frucht der Pflanze.

7. Verwendung nach einem der Ansprüche 1 bis 4, wobei die Zubereitungen Saponine enthalten, die ausgewählt sind aus der Gruppe, die gebildet wird von Arganin A, Arganin B, Arganin C, Arganin D, Arganin E, Arganin F, Arganin G, Arganin H, Arganin J und Misaponin A.

8. Verwendung nach einem der Ansprüche 1 bis 4, wobei die Zubereitungen den Saponine enthaltenden Pflanzenextrakt in Mengen von 0,01 bis 25 Gew.-% bezogen auf die Endzubereitungen enthalten, mit der Maßgabe, dass sich die Mengenangaben mit Wasser und gegebenenfalls weiteren Hilfs- und Zusatzstoffen zu 100 Gew.-% addieren.

## Claims

1. Use of preparations that contain saponins from an extract of the plant Argania spinosa in cosmetic care compositions for hair and/or skin.

2. Use according to Claim 1 for improving the combability, the shine, the volume and the flexural strength of hair.

3. Use according to Claim 1 as slimming and anticellulite care compositions for the skin.

4. Use according to Claim 1 in sunscreens.

5. Use according to one of Claims 1 to 4, where the extract is obtained by extraction from parts of plants, selected from the group consisting of the leaves, the roots, the stem, the bark, the flowers, the fruits, the fruit flesh and the seeds.

6. Use according to one of Claims 1 to 4, where the extract is obtained by extraction of the seeds and/or the defatted seeds from the fruit of the plant.

7. Use according to one of Claims 1 to 4, where the preparations comprise saponins selected from the group consisting of arganine A, arganine B, arganine C, arganine D, arganine E, arganine F, arganine G, arganine H, arganine J and misaponine A.

8. Use according to one of Claims 1 to 4, where the preparations comprise the plant extract containing the saponins in amounts of from 0.01 to 25% by weight, based on the final preparation, with the proviso that the quantitative data add up to 100% by weight with water and optionally further auxiliaries and additives.

## Revendications

1. Utilisation de préparations contenant des saponines issues d'un extrait de la plante Argania spinosa dans des agents de soin cosmétiques pour les cheveux et/ou la peau.

2. Utilisation selon la revendication 1 pour améliorer l'aptitude au coiffage, le brillant, le volume et la résistance à la flexion des cheveux.

3. Utilisation selon la revendication 1 en tant qu'agent de soin amincissant et anti-cellulite pour la peau.

4. Utilisation selon la revendication 1 dans des agents de protection solaire.

5. Utilisation selon l'une des revendications 1 à 4, l'extrait étant obtenu par extraction de parties de plantes sélectionnées parmi le groupe formé des feuilles, des racines, des tiges, de l'écorce, des fleurs, des fruits, de la pulpe de fruit et des graines.

6. Utilisation selon l'une des revendications 1 à 4, l'extrait étant obtenu par extraction des graines et/ou des graines dégraissées issues du fruit de la plante.

7. Utilisation selon l'une des revendications 1 à 4, les préparations contenant des saponines sélectionnées parmi le groupe formé de l'arganine A, l'arganine B, l'arganine C, l'arganine D, l'arganine E, l'arganine F, l'arganine G, l'arganine H, l'arganine J et la misaponine A.

8. Utilisation selon l'une des revendications 1 à 4, les préparations contenant l'extrait de plante contenant les saponines dans des quantités comprises entre 0,01 et 25 % en masse, sur la base des préparations finales, à la condition que les indications de quantité soient complétées avec de l'eau et éventuellement d'autres agents auxiliaires et additifs à 100 % en masse.
